# EUROPEAN PATENT APPLICATION

(11) **EP 1 547 674 A1**
(43) Date of publication of application: **29.06.2005**
(21) Application number: 03029798.0
(22) Date of filing: 23.12.2003
(51) Int. Cl.: B01J 13/00

(54) **Method for producing colloidal nanoparticles**

(71) Applicant: MediGene Oncology GmbH, 82152 Planegg/Martinsried (DE)
(72) Inventor: The designation of the inventor has not yet been filed
(74) Representative: Weiss, Wolfgang, Dipl.-Chem. Dr.

(57) **Abstract**

The invention relates to an improved method for producing homogenous colloidal nanoparticles, preferably liposomes and the use of a compounder for the preparation of these.

## Description

Liposomes are small, spherical vesicles composed primarily of various types of lipids, phospholipids or other lipophilic components. The lipid components normally form a bilayer, wherein the polar end of the amphiphile is in contact with the surrounding solution, which is typically an aqueous medium. The non-polar, amphiphilic end of the amphiphile is in contact with another non-polar, amphiphilic end of another amphiphile thereby forming the lipid bilayer. Depending on the type of amphiphiles used, the liposome membrane can be classified according to their outer charge into net neutral, negatively and positively charged membranes. Liposomes are developed for many therapeutic and diagnostic applications. Among others they are used to deliver molecules which are not sufficiently soluble in water. These lipophilic molecules are incorporated into the liposome bilayer or have been chemically linked to the lipid bilayer.

Liposomes have widely been used for the loading of biologically active ingredients and hydrophilic substances might be encapsulated in the aqueous internal compartment of a liposome or, in case of a lipophilic substance, incorporated in the lipidic bilayer itself. In case of a dermal application of liposomes, active ingredients or substances might be transported into deeper regions of the skin due to the amphiphilic properties of liposomes.

The fields of liposome applications are widespread, including e.g. parenteral, oral, topical, and inhalative formulations. Advantages of liposome application include the possibilities of a controlled or sustained release, increased drug delivery as well as reduced side effects as the drug delivery might be better localized and many other effects. Thus, liposomes are a promising tool to overcome common obstacles in targeted substance application.

There are lots of variations in liposome morphology such as the number of bilayers, size, surface charge or modification, as well as lipid composition and preparation method that can be adjusted to obtain an ideal drug delivery system.

Among the various types of liposomes, mostly those are regarded favourable, which have a small to intermediate vesicle size such as from about 50 to 300 nm diameter combined with a unimodal size distribution. A uniform size distribution provides reproducible and well characterizable liposome populations.

Phospholipids with membrane-building properties have the ability - upon dispersion in an appropriate medium - to swell, hydrate and to form concentric bilayers. These multilamellar concentric bilayers are separated by layers of aqueous medium. These vesicles are called multilamellar lipid vesicles (MLV). Their diameter typically ranges from 300 nm up to 2 µm. The MLV have first been described by Bangham (see Bangham, A. D., et al. Biol. 8 (1964), pages 660 ff.).

Liposomes can also form vesicles consisting of only one bilayer; they are commonly referred to as unilamellar vesicles. They form one single spherical lipid bilayer that surrounds the aqueous medium. If these unilamellar vesicles have an average diameter of about 30 to 300 nm, they are called small unilamellar vesicles (SUV), while larger unilamellar vesicles can have diameters in the range of 300 nm up to 1 µm.

For the preparation of liposomes, numerous methods have been described. An overview of the most common methods for the preparation of liposomes and adherend facts and methods concerning lipids is given in "Liposome Technology", ed. G. Gregoriadis, CRS Press Inc., Boca Raton, Florida, Vol. I, II and III (1993). Most of these methods, however, have focused on the use of an organic solvent or detergent in which the membrane forming lipids as well as active ingredients are completely dissolved or solubilized prior to dispersion in an aqueous medium. The solubilization step further ensures complete mixing of the liposomal components. Typically the preparation of liposomes is performed in at least 2 or more steps. A first step is the dissolving of the lipids in a suitable solvent, followed by a further processing of either the solution or of a lipid film obtained by the evaporation of the organic solvent. (See Bangham, A. D., et al., Meth. in Membrane Biol.1, pages 1 - 68). The solvent is usually removed under vacuum by rotary evaporation. When evaporated, the residual lipids form a thin film on the wall of the container or the round-bottom flask. An aqueous medium that may contain hydrophilic material is then added to the lipid film. By mild agitation of the round-bottom-flask resp. container the lipid film is hydrated, forming large multilamellar vesicles (MLV). Up to this point the preparation technique is called the film method. If smaller vesicles are desired further process steps have to be applied.

The size of the liposomal vesicles can be determined by quasi-elastic laser light scattering (QELS) (Bloomfeld, et al., Ann. Rev. Biophys. Bioeng., 10: 421 - 450 (1981)) or photon correlation spectroscopy (PCS). The average liposome diameter in nm is hereby determined and the size distribution mode can be observed. The size distribution is given by the so-called polydispersity index - a figure between 0 and 1, with an increasing number describing a broadening size distribution. Thus monodisperse liposome populations (indicated by a polydispersity index of 0 up to 0.2) can be detected as well as a polydisperse size distribution figured by a number of > 0.2. A polydispersity index below 0.2 should be pursued as it assures a reproducible liposome quality amongst other factors.

A prominent method for downsizing of MLV is the extrusion technique (see Hope, MJ et al., Reduction of liposome size and preparation of unilamellar vesicles by extrusion techniques. In: "Liposome Technology", ed. G. Gregoriadis, CRS Press Inc., Boca Raton, Florida, Vol. I, page 123 (1993)). The method is performed by sequential filtration of the liposome (MLV) dispersion through filters of defined pore size. Usually polycarbonate membranes are used for this purpose as they have a clearly certified - assured by their manufacturing process - pore size, but also other filters might be used as for example cellulose acetate filters originally designed for the sterile filtration of solutes. The suspension is cycled through the membrane one or several times until the desired liposome size is achieved. The liposomes may be extruded using successively smaller pore-sized membranes to yield a gradual reduction in liposome size. Usually a relatively well-defined liposomal size distribution is obtained.

A variety of alternative methods are known to a person skilled in the art for reducing the size of MLV in order to obtain SUV. Another common method for the production of SUV is sonication. The sonication of an aqueous dispersion of phospholipids either by bath or by probe sonication results in a progressive size reduction down to SUV of less than about 50 nm in diameter, as described by Papahadjopoulos, et al., Biochim et Biophys Acta 135:224 - 238 (1968).

SUV can also be prepared by the ethanol-injection-technique described by Batzri, et al., Biochim et Biophys Acta 298: 1015 - 1019 (1973) or the ether injection technique of Deamer, et al., Biochim et Biophys Acta 443: 629 - 634 (1976). These methods are performed by the rapid injection of an organic solution of lipids into an aqueous medium or a buffer solution under gentle stirring. The obtained dispersion contains unilamellar liposomes of which the size might be influenced up to a certain extend by the conditions chosen for the ethanol injection (e.g. lipid concentration, injection speed, etc.) or by consecutive extrusion steps.

A further method for the production of SUV is given by Wedder, et al., in "Liposome Technology", ed. G. Gregoriadis, CRS Press Inc., Boca Raton, Florida, Vol. I, Chapter 7, pages 79 - 107 (1984). The method described is called detergent removal method and is based on the removal of a detergent in which the lipids and additives had been solubilized forming mixed micelles.
The reversed phase evaporation technique involves the formation of a water-in-oil emulsion of lipids in an organic solvent and an aqueous buffer solution. When the organic solvent is removed by the use of pressure the lipids are converted to large unilamellar vesicles (LUV). The method is described by Papahadjopoulos, et al., US Patent 4,235,871.

In the US patent No. 4,016,100 (Suzuki, et al.) a method is described to encapsulate material in vesicles prepared by freezing and thawing of an aqueous phospholipid dispersion.

Rotor-stator homogenization or liquid high-pressure homogenization are suitable for the preparation of liposomes on a larger scale. They comprise the recirculation of a raw lipid dispersion through a small shear valve using moderate to high pressures (e.g. 100 bar up to 1400 bar). The occurring phenomena during the homogenization process are collision and cavitation in addition to the mere shear process. Although liquid high pressure homogenizers, which include, for example, microfluidizers, are very well suited for the preparation of liposomes, they have the disadvantage that there is a high energy impact that acts on the lipids and active ingredients processed by high pressure homogenization. As a consequence substances sensitive to shear stress (e.g. proteins..) cannot be handled by high pressure homogenization. The typical liposome population obtained by high-pressure homogenization is of vesicle diameters between 50 and 300 nm mostly showing polydisperse size distribution matters, indicating a broad size distribution. A broad size distribution however, is generally seen as disadvantageous as it leads to poorly characterizable and hardly reproducible liposome dispersions.

The known methods, however, have tremendous technical, economical and environmental drawbacks.
Current methods for the preparation of liposomes are mostly batch processes. Especially the well-established film-method cannot be scaled up exceeding a certain volume. There is nearly no manufacturing technique available that allows a continuous liposome preparation nor have attempts for up-scaling the manufacturing processes been quite successful. Thus, there is a high need for improvements concerning manufacturing techniques working in a more continuous matter and leaving those poorly characterizable batch-sized methods behind.

Additionally other problems have to be faced as for example the residual solvents that may remain up to a certain extend within the final liposomal product. The removal of organic solvent from the thin lipid film prepared during the "film method" is often difficult to accomplish and thus incomplete. As a result the final liposome dispersion might contain traces of residual solvent, which is potentially toxic, and in the case of certain solvents (e.g. chloroform, dichloromethane, acetone) even carcinogenic. So medical application of liposomes prepared using organic solvent needs specific monitoring as far as solvent traces are concerned. From the manufacturing point of view the film method does not fulfill the needs of a simple, easily scalable and safe preparation technique.

Reproducibility is asked for all applications of liposomes, but when focusing on parenterally administered liposomes, it becomes a basic requirement for any approval of the final product. Techniques employing high shear forces as for example high-pressure homogenization do not meet these demands mainly due to the wide size distributions of the liposome dispersions obtained thereof. Also ultrasonic preparation techniques often lead to liposome populations with a broad size distribution. In the case of an i.v. injection of liposomes their size should be limited. A size of about 50 nm to 200 nm and a sufficient size homogeneity is regarded as necessary. (see: Huang, L. "Size homogeneity of a liposome preparation is crucial for liposome distribution in vivo", J. Liposome Res. 2, 57 - 66, 1992)

Thus, the problem underlying the present invention is to provide an improved method of producing homogenous colloidal nanoparticulate systems such as micelles, liposomes or lipid nanospheres which is particularly suitable for large scale production.

The solution to the above problem is achieved according to the invention by providing the embodiments characterized in the claims. The invention relates to a method for producing homogenous colloidal nanoparticles, comprising the steps of
a) extruding a composition comprising at least one amphiphilic component by means of a compounder,
b) dispersing the extruded composition of step a) in an aqueous medium,
c) optionally homogenizing the preparation of step b) at least once and/or
d) optionally sterile filtrating the preparation of step b) or c),
wherein optionally at least one active agent is present in the composition of step a) and/or in said aqueous medium of step b).

The composition of step a) might further optionally comprise an aqueous medium, an organic solvent or a detergent.

Extrusion of polymers by means of a "compounder" is well known in the art and used for mixing different polymers (blending/alloying) or compounding of additives. For blending amphiphilic components such as fats, oils, waxes, amphiphiles and the like, optionally together with an active agent however, compounder were not used so far.

Surprisingly it was found, that by the aid of a compounder colloidal nanoparticles can be produced in a quick and easy way. Thereby, amphiphilic components, optionally together with at least one active agent can be mixed to a very high degree (e. g. for liposomes with a FRET of about 80% to about 100% of reference liposomes prepared by the film method) without dissolving the aforementioned components in an organic solvent, mixing them with an aqueous medium or a detergent or exposing them to severe conditions such as a high pressure homogenizing process. Thereby the amphiphilic component(s), optionally together with an active agent, are simply weighed together and brought into a compounder. The latter can be designed as a cylinder and plunger system or a screw-extruder as known from blending polymers. The amphiphilic component(s) can subsequently be extruded by applying a pressure force to the plunger. The resulting mass can be dispersed in a suitable aqueous medium to prepare colloidal nanoparticles, such as micelles, liposomes, lipid nanospheres, emulsions or gels.

The use of a compounder is also suitable for preparing homogenous colloidal nanoaggregates comprising of only a single amphiphilic component. Thereby a time and energy consuming or even harsh step such as within the film method, the organic solution injection method or high pressure homogenization is avoided. Thus, in preferred embodiments of the present invention step a) of the novel method can be performed without the presence of an aqueous medium, an organic solution and/or a detergent.
The resulting colloidal nanoparticles are of very high homogeneity and have a high FRET rate (for liposomes the rate is about 80 % to about 100 % of reference liposomes prepared by the film method as a standard) while all disadvantages of other methods, particularly the film method are avoided. Reference liposomes prepared by the film method are thereby equally distributed in terms of the Pl value or of the same size (of about 150 nm).

At least one active agent can thereby be present in both cases either during step a) and/or step b) of the inventive method e. g. one single amphiphilic component plus one or more active agents may be extruded by the compounder.

Dispersing of step b) of the inventive method can be performed by simple stirring the resulting mass of step a) in an aqueous medium.

A subsequent homogenization step c) of the present invention is preferably performed by extrusion and/or filtration through a membrane filter device (e.g. a Lipex® thermobarrel extruder) using extrusion membranes of defined pore size and mostly preferred by extrusion through a membrane with a pore size of about 200 nm under pressure. Membranes with other pore sizes such as 50 nm, 100 nm, 150 nm, 400 nm well known in the art may be used as well. Filtration through membrane filters maybe performed by filtration through membranes composed of PVDF, PES, Nylon-filters but also other materials may be used if defined to be suitable. Preferably polycarbonate filters are used. Pore size of membranes shall be in the range of about 100 nm to 400 nm, but pore size is not limited to the sizes mentioned. Different materials and different pore sizes maybe combined in a way to obtain a solution which maybe processed by a sterilizing grade filtration.
Homogenization can further be achieved by ultrasonic processing (e.g using a Branson® sonifier) of the dispersion but is not preferred.
Depending on the pore size or ultrasonic processing time the size of the resulting colloidal nanoparticles ranges from about 20 nm to about 1000 nm, preferably from about 50 nm to about 500 nm and more preferably from about 100 nm to about 300 nm.

The colloidal nanoparticles can be sterilized according to step d) e. g. by means of sterile filtration with 0.2 µm sterile filters. (e.g.: Durapore® (PVDF) Fa. Millipore, Eschborn, Germany).

A suitable aqueous medium according to step a) or b) of the present invention comprises water and optionally at least one suitable additive such as a hydrophilic, bio compatibel solvent like propylen, gycol, glyerol, PEG or others which is completely dissolved in water. Such additives may be also buffers or their individual components, cryoprotectants or further stabilizing agents such as antioxidants. A cryoprotectant can be selected from a sugar or an alcohol or a combination thereof such as trehalose, maltose, sucrose, glucose, lactose, dextran, mannitol or sorbitol in the range of up to about 20 % (m/v).

In a preferred embodiment the organic solution step a) comprises an organic solvent preferably selected from biocompatible solvents which ban be accepted after dilution into the final applicable form according to the Guidelines of solvents of drug products, preferably ethanol. Further examples are methanol, propanol, isopropanol, ethylene glycol, propylene glycol, t-butanol, glycerol, sorbitol, solketal, n-pyrrolidon, soluphor, tetrahydrofuran, ethylacetate, methylenechloride or a mixture of these solvents.

In a further preferred embodiment a suitable detergent can be selected from Tweens, Pluronics, poloxamers, Cremophors, lecithins, Solutol HS15, SDS (sodium dodecylsulfate) or any other physiologically acceptable surfactant, preferably lecithins.

In a more preferred embodiment, the inventive method is performed without the presence of an aqueous medium, organic solvent or detergent in step a).

Suitable amphiphilic components are selected from amphiphiles such as fats, oils, lipopolysaccharides, lipoproteins, sterols or lipids such as cholesterol or phospholipids, lysolipids, lysophospholipids, sphingolipids or pegylated lipids, but also waxes with a positive, negative or neutral net change. Also partially esterified derivatives of glycerol like monoglycerides, -diglycerides, triglycerides and their derivatives can be selected. Anionic or neutral amphiphiles can be selected from sterols or lipids such as cholesterol, phospholipids, lysolipids, lysophospholipids, sphingolipids or pegylated lipids with a negative or neutral net change. Useful anionic and neutral lipids thereby include: Phosphatidic acid, phosphatidylserine, phosphatidylglycerol, phosphatidylinositol (not limited to a specific sugar), fatty acids, sterols containing a carboxylic acid group, cholesterol, 1,2-diacyl-sn-glycero-3-phosphoethanolamine, including but not limited to dioleoyl (DOPE), 1,2-diacyl-glycero-3-phosphocholin and sphingomyelin. The fatty acids linked to the glycerol backbone are not limited to a specific length or number of double bonds. Phospholipids may also have two different fatty acids. In a preferred embodiment the neutral amphiphile is diacylphosphatidylcholine.

Useful cationic lipids thereby include:
DDAB, dimethyldioctadecyl ammonium bromide; N-[1-(2,3-dioleoyloxy)propyl]-N,N,N-trimethyl ammonium (DOTAP); 1,2-diacyloxy-3-trimethylammonium propanes, (including but not limited to: dioleoyl, dimyristoyl, dilauroyl, dipalmitoyl and distearoyl; also two different acyl chain can be linked to the glycerol backbone); N-[1-(2,3-dioloyloxy)propyl]-N,N-dimethyl amine (DODAP); 1,2-diacyloxy-3-dimethylammonium propanes, (including but not limited to: dioleoyl, dimyristoyl, dilauroyl, dipalmitoyl and distearoyl; also two different acyl chain can be linked to the glycerol backbone); N-[1-(2,3-dioleyloxy)propyl]-N,N,N-trimethylammonium chloride (DOTMA); 1,2-dialkyloxy-3-dimethylammonium propanes, (including but not limited to: dioleyl, dimyristyl, dilauryl, dipalmityl and distearyl; also two different alkyl chain can be linked to the glycerol backbone); dioctadecylamidoglycylspermine (DOGS);, 3□-[N-(N',N'-dimethylaminoethane)carbamoyl]cholesterol (DC-Chol); 2,3-dioleoyloxy-N-(2-(sperminecarboxamido)-ethyl)-N,N-dimethyl-1-propanaminium trifluoro-acetate (DOSPA); □-alanyl cholesterol; cetyl trimethyl ammonium bromide (CTAB); diC14-amidine; N-*tert*-butyl-N'-tetradecyl-3-tetradecylaminopropionamidine; 14Dea2; N-(alpha-trimethylammonioacetyl)didodecyl-D-glutamate chloride (TMAG); O,O'-ditetradecanoyl-N-(trimethylammonioacetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butanediammonium iodide; 1-[2-(acyloxy)ethyl]2-alkyl(alkenyl)-3-(2-hydroxyethyl)-imidazolinium chloride derivatives as described by Solodin et al. (1995) Biochem. 43:13537-13544, such as 1-[2-(9(Z)-octadecenoyloxy)ethyl]-2-(8(Z)-heptadecenyl-3-(2-hydroxyethyl)imidazolinium chloride (DOTIM), 1-[2-(hexadecanoyloxy)ethyl]-2-pentadecyl-3-(2-hydroxyethyl)imidazolinium chloride (DPTIM), 2,3-dialkyloxypropyl quaternary ammonium compound derivatives, contain a hydroxyalkyl moiety on the quaternary amine, as described e.g. by Felgner et al. [Felgner et al. *J. Biol. Chem.* 1994, 269, 2550-2561] such as: 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimetyl-hydroxypropyl ammonium bromide (DORIE-HP), 1,2-dioleyloxypropyl-3-dimethyl-hydroxybutyl ammonium bromide (DORIE-HB), 1,2-dioleyloxypropyl-3-dimethyl-hydroxypentyl ammonium bromide (DORIE-Hpe), 1,2-dimyristyloxypropyl-3-dimethyl-hydroxylethyl ammonium bromide (DMRIE), 1,2-dipalmityloxypropyl-3-dimethyl-hydroxyethyl ammonium bromide (DPRIE), 1,2-disteryloxypropyl-3-dimethyl-hydroxyethyl ammonium bromide (DSRIE); cationic esters of acyl carnitines as reported by Santaniello et al. [US5498633]. In a preferred embodiment the cationic lipid is selected from a quaternary ammonium salt such as N-[1-(2,3-diacyloxy)propyl]-N,N,N-trimethyl ammonium, wherein a pharmaceutically acceptable counter anion of the quaternary amino compound is selected from the group consisting of chloride, bromide, fluoride, iodide, nitrate, sulfate, methyl sulfate, phosphate, acetate, benzoate, citrate, glutamate or lactate. In a more preferred embodiment the cationic lipid is DOTAP.

Unless defined otherwise, all technical and scientific terms used in this specification shall have the same meaning a commonly understood by persons of ordinary skill in the art to which the present invention pertains. "Active agent" as used herein includes biologically active agents and means any molecule that acts as a beneficial or therapeutic compound. Examples of active agents by means of illustration and not limitation are diagnostics, anti-cancer-agents, contrasting media, fluorescent markers and drugs for any other indication, including protein and peptide drugs etc. Further, the term "active agent" can be used more broadly and means any chemical compound or material that is desired to be applied, administered or used in colloidal nanoparticles such as liposomes, and can include, by way of illustration (not to be meant limiting): imaging agents, dyes and fluorescent markers and so on. The active agent can thereby be either hydrophilic or lipophilic. Specific examples for preferred active agents are selected from taxanes, from other agents interacting with microtubuli such as epothilones, discodermolide, laulimalide, isolaulimalide, eleutherobin, colchicines and derivatives thereof, vinca alkaloids such as vinorelbine, from platinum complexes such as oxaliplatin, from camptothecins, from anthracyclines such as doxorubicin or from statins (e.g., lovastatin).

"Colloidal nanoparticels" refers to any colloidal particulate system such as micelles, liposomes, lipid nanospheres, nanocapsules or emulsions.

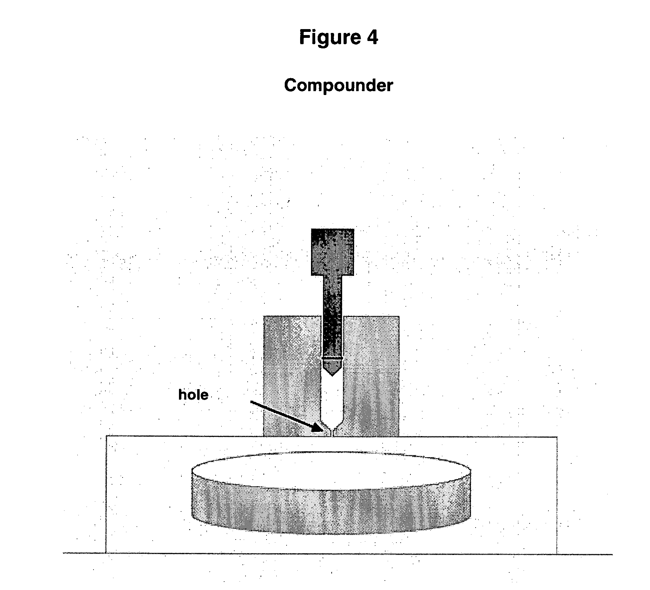

"Compounder" as used herein refers to an apparatus as depicted in Figure 4, or a single or double-screw extruder as e.g. provided by ThermoHaake, Karlsruhe, Germany. The compounder can be designed as a batch or a continuous extruder. For laboratory purposes the compounder can be designed as a cylinder and plunger system wherein the cylinder has an open bore of about 0.2 mm to about 1.4 mm, preferably about 0.4 mm to about 1.2 mm, most preferably 0.8 mm at the lower end. Single amphiphiles or a lipid mass can be extruded through this open end by applying pressure force to the plunger. Extrusion can also be carried out by a screw-extruder through an extrusion plate with one ore more bores. The extruder can be constructed in a way that allows recirculation of the extrudate within the extrusion chamber before extrusion through the nozzle. The compounder works with a low pressure of 0,2 bar to 100 bar, preferably 0,5 bar to 10 bar and most preferably 1 to 6 bar and at a temperature of about 5°C to about 100°C, preferably 20°C to about 70°C and most preferably of about 25°C to about 50°C. A scale up of the compounder to any size is possible.

"Drug" as used herein refers to a pharmaceutically acceptable pharmacologically active substance, physiologically active substance and/or a substance for diagnostic use.

"Extruder" refers to an apparatus which forces liquid dispersions, typically aqueous liposome dispersions under pressure through filters, such as polycarbonate filters or bacteria retentive filters suitable for sterile filtration, with a defined pore size.

"High pressure homogenization" refers to an apparatus that consists of a cylinder piston pump and a homogenizing valve through which a liquid or dispersion is forced under high (100 up to 2000 bars) pressure. It is a characteristic of the high pressure homogenizer that the expansion of the pressureized dispersion immediately after passing through the opening of the valve leads to extremely efficient shear forces and consecutively to reduction of particle sizes and increase in the degree of dispersion. An example for an high-pressure-homogenizer is the APV Gaulin Micron Lab 40™ high pressure homogenizer (APV-Gaulin, Lübeck, Germany), that works according the above described principles. It is possible to homogenize MLV dispersions obtained according to the established film-method or even to homogenize raw lipid-dispersions.

"Homogenization" refers to a physical process that achieves a higher level of dispersion of a nanoparticulate system in an aqueous medium/ two phases in an emulsion. One example is high-pressure homogenisation.

"Homogenous" in the context of the present invention means even molecular distribution of different compounds within a colloidal particulate system. Not only is the dispersion homogeneous with respect to the even distribution of its compounds in different aliquots of the dispersion but homogeniety is also achieved on a molecular level within the single colloidal particles. When colloidal particles are composed of different molecular entities, the quantitative composition from particle to particle is relevant for homogeneity. Particularly homogenous liposomal preparations refer to preparations with a homogeneity determined by FRET which are at least as good as preparations produced by the lipid film method, i. E. the relative FRET is at least about 80 %, preferably about 80 % to about 100 % compared to FRET observed for preparations produced with the film method.

"Lipid" refers to its conventional sense as a generic term encompassing fats or lipids, which are insoluble in water. Lipids compose fats, fatty oils, essential oils, waxes, steroid, sterols, phospholipids, glycolipids, sulpholipids, aminolipids, chromolipids, and fatty acids. The term encompasses both naturally occurring and synthetic lipids. Preferred lipids in connection with the present invention are: steroids and sterols, particularly cholesterol, phospholipids, including phosphatidyl and phosphatidylcholines and phosphatidylethanolamines, and sphingomyelins. Fatty acids can have about 12-24 carbon chains in length, containing up to 6 double bonds, and linked to the backbone, and they can be different (asymmetric), or may only have 1 fatty acid chain, e.g., lysolecithins. Mixed formulations are also possible, particularly when non-cationic lipids are derived from natural sources, such as lecithins (phosphatidylcholines) purified from egg yolk, bovine heart, brain, or liver, or soybean.

The advantages of the present invention are as follows:

The inventive method is quick and simple. Amphiphilic components can be compounded and thereafter used to prepare colloidal nanoparticles without the need of an organic or aqueous medium or a detergent in the amphiphilic phase. The method can easily be adapted to a large scale production.

The preparation process according to the invention has very mild conditions, e.g. no elevated temperatures occur, nor is sonication or shear stress with high impact forces necessary for preparing the mixture of components. Thus, components can be processed that are sensitive to heat and or ultrasonic stress and that tend to hydrolyse in presence of water and /or heat.

In contrast to conventional manufacturing techniques, like, for example, the ethanol-injection-technique, [see: Batzri, et al., Biochim et Biophys Acta 298: 1015 - 1019 (1973)] colloidal nanoparticles obtained by the novel method of the invention show a very high degree of mixing. When for example a pre-liposomal lipid dispersion is prepared by merely dispersing (lyophilised as well as non-lyophilised) lipids in an aqueous medium, the level of lipid mixing is not as high as if lipids are pre-homogenized by means of the compounder described herein. The process of pre-homogenizing lipids with the compounder before using in order to prepare liposomes, leads to a much higher level of lipid mixing throughout the liposomal bilayer in contrast to merely mechanical dispersing lipids in aqueous medium, for example by stirring the solute using a magnetic stirrer. Established preparation techniques do not deliver a comparably high degree of lipid mixing throughout the liposomal bilayer, except when organic solvents are involved in the preparation protocol.

The compounded mass can be dispersed in an aqueous medium and subjected to a sizing procedure in order to obtain nanoparticles of defined characteristics as expressed in the particle diameter and/or polydispersity index. A further advantage, especially for liposomes designed for parenteral use, is a clearly defined size distribution of the liposome populations obtained by using the novel method and a subsequent homogenization step since polydispersity indices of liposomes which are prepared by the inventive method always indicate a narrow size distribution and thus very well characterizable liposome populations. These liposomes have a high quality since a subsequent sterilization process does not alter the characteristics of the liposome dispersion as expressed in the vesicle size (Zₐᵥₑ) and polydispersity index. Thus the high quality of the liposomes prepared using the apparatus according the invention is not declined by a consecutive sterilization step. Thus, the easy adaptable size as well as the narrow size distribution of the vesicles produced by the novel preparation technique is a clear advantage as compared to conventional manufacturing techniques where the liposome size can hardly be influenced.

Of special advantage is the option, that the active agent - if desired - might be added right in the beginning of the preparation protocol, that is to say, the chemicals / drugs might be admixed to the amphiphilic components) used for liposome preparation before being transferred to the compounder. In case of lipophilic chemicals / drugs this mixing of the ingredients right in the beginning, guarantees an optimal distribution of the chemicals / drugs within the cationic nanoparticles, e. g. liposomes and simplifies the whole operation process as a second weighing step can be avoided. If desired, the drug loaded lipid-mass can be stored as intermediate and aliquots of this intermediate might be supplied to a hydration process.

The method of the invention permits the production of a wide variety of different colloidal nanoparticles that can easily be adapted to the particular requirements of the product formula and the application for which the final product is intended.

Thus, the novel technique disclosed herein delivers colloidal nanoparticles which are mixed to a very high degree and which are considerably homogeneous even on molecular level.

The aforementioned degree of mixing of the single components on molecular level can be monitored using the Fluorescence Resonance Energy Transfer (FRET) technique. Fluorescence Resonance Energy Transfer (FRET) is an interaction between the excited states of electrons in two dye molecules. The strength of the interaction is depending on the distance of the molecules. Excitation from a donor dye molecule is transferred to an acceptor dye molecule if a proximity of typically 10 - 100 Å is given. A further primary condition for FRET is an overlap of the fluorescence emission spectrum of the donor molecule with the absorption spectrum of the acceptor molecule. Thus, FRET is a valuable technique for investigating phenomena e. g. in liposome bilayers where proximities of lipid molecules and their distribution within the liposomal membrane can be studied when fluorescently labelled lipid molecules are incorporated in the lipid bilayers of liposomes. Fluorescence intensities are directly related to the efficiency of energy transfer from the donor to the acceptor dye molecule. A low fluorescence intensity thus indicates a low level of lipid mixing of the fluorescently labelled lipids in the bilayers of the liposomes, as the probability of proximity of different lipid molecules is statistically low when mixing is incomplete.

The inventive method can preferably be used to produce liposomes. These are characterized by having a polydispersity index (PI) of below about 0.4, preferably of below about 0.3, more preferably of below about 0.25 and most preferably of below about 0.2.

Liposomes of the present invention can be anionic, cationic or neutral. Preferred liposomes are cationic and comprise DOTAP, DODAP, analogues of DOTAP or DODAP or any other cationic lipid in an amount of at least about 30 mol% of the total liposome-forming lipid, preferably of at least at least about 40 mol%, more preferably of at least about 50 mol%.

The method of the present invention permits the production of a wide variety of different colloidal nanoparticles that can easily be adapted to the particular requirements of the product formula and the application for which the final product is intended. Emulsions or gels can also be prepared by the inventive method as disclosed. In case of a pharmaceutical use, typically an active agent is loaded within the colloidal nanoparticles. Although the products of the invention are particularly well suited for pharmaceutical use, they are not limited to that application and may also be designed for any industrial or cosmetical use, for imaging studies, food and agricultural applications, where lipid vesicle encapsulation and administration of compounds and materials is warranted or useful. Thereby included are dietary supplements, vitamine formulations, antioxidative formulations ( tocopherols) and cosmetic formulations.

In a preferred embodiment the present invention contemplates the manufacture of colloidal nanoaggregates without an active agent. Particularly "empty liposomes" are currently used in cosmetic preparations as well as placebo materials in clinical trials of therapeutically applied liposome formulations. Typically, an active agent is loaded either in the aqueous core of liposomes or within the lipid bilayer of the lipid vesicle.

Thus, there are various alternative options to produce colloidal nanoaggregates comprising at least one, optionally more than one active agent(s), especially to load active agents into liposomes. The wide variety of altering preparation protocols allows to ideally fulfil the needs of the desired product and to follow specific (physicochemical) properties of the chemicals involved in the formulation. Firstly, it is possible to add an active agent before beginning of the preparation process by adding the substance to the amphiphilic component(s) that are intended to be used (that is in step a) of the inventive method). Secondly, an active agent can be added upon dispersion and thus hydration of the mass obtained by the compounder, colloidal nanoaggregates such as liposomes are formed which are capable of entrapping an aqueous solute (that is step b) of the inventive method). Loading of one or more active agent(s) e. g. into the liposomes can be accomplished during step b) of the inventive method when the mass produced by using the compounder in step a) is hydrated with or dispersed in a suitable aqueous medium, such as water, water miscible solvents, saline, or an appropriate buffer, containing a desired compound for encapsulation.

As a further possibility it is also possible to add an active agent after empty liposomes have been prepared, using techniques like remote loading (e.g. as established for the active agent Doxorubicin). Another method employs dehydration-rehydration cycles that even enables to load empty liposomes prepared according to the invention with an active agent that is trapped upon the rehydration processes. Acting similarly, a retroactive loading of liposomes might be performed using freeze-thaw cycles: here a chemical or a drug can be incorporated into initially prepared liposomes by repeatedly freezing and thawing the liposomes in presence of a solute containing the desired chemical or drug.

These methods are only meant to be illustrative and are not limiting. Consequently they can be subjected to any modification desired. Even temperature-sensitive materials / active agents or materials / active agents that cannot be processed using harsh shear forces can be used with the inventive method. Thus, the novel preparation process allows to fulfil all needs due to physicochemical properties of the chemicals used. It allows to add the desired component(s) and active agent directly before adding them to the compounder and thus by-passing any temperature stress for the materials involved. The preparation method further allows to add the desired active agent during the hydration process of the mass obtained by using the compounder. The preparation method also allows to add the desired active agent after e. g. empty liposomes have been prepared according the invention, employing techniques such as remote loading or freeze-thaw cycles.

In summary, the preparation method according the invention allows to add the desired active agent whenever it is considered as reasonable during the whole preparation procedure and might be modified to the greatest extend possible.

The versatility of the present invention is illustrated, but not limited, by the following examples.

### Examples

The following examples illustrate the invention and are non-limiting embodiments of the invention claimed below.

Temperatures of all Examples are given in degrees centigrade.

### Example 1

| | |
|---|---|
| 80 mgs | egg-phosphatidyl-choline (Egg-PC) |
| 10 ml | glucose 5% |

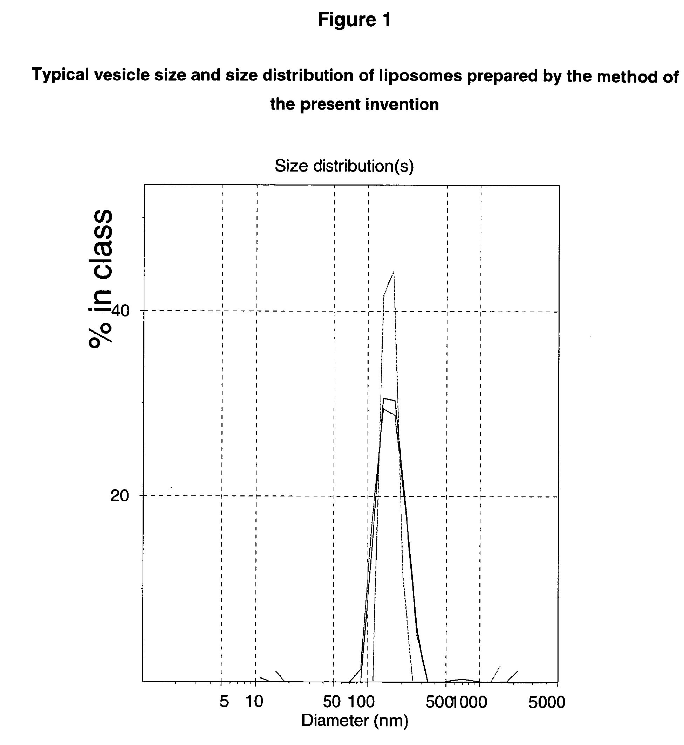

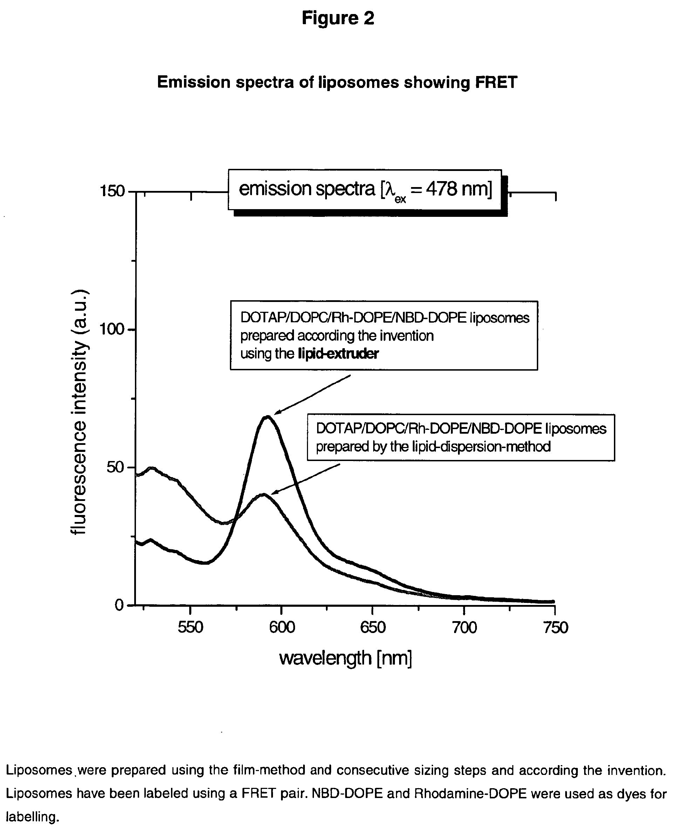

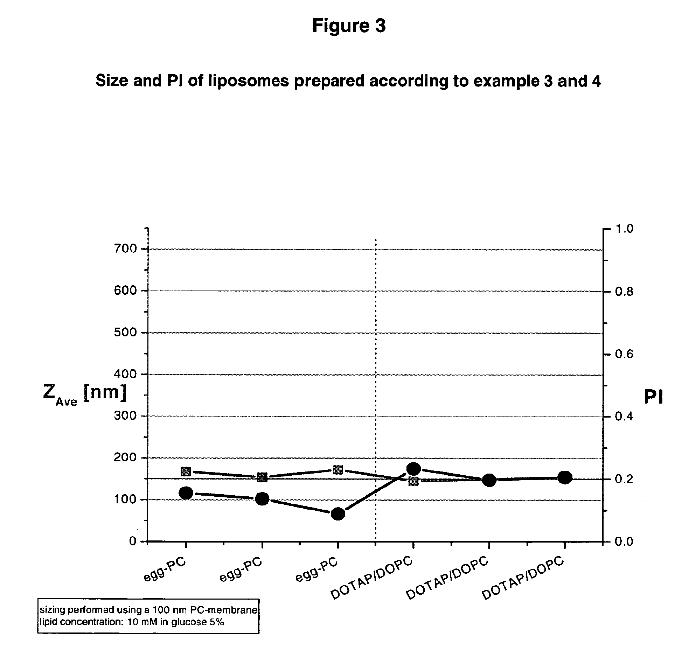

The powdered egg-PC is homogenized at room temperature 3 times (3 cycles) using the apparatus relating to the invention as depicted in Fig. 3, without using any solvent or solubilizer. The obtained lipid-mass is dispersed by stirring in aqueous medium (Glucose 5%) and allowed to hydrate and swell for approximately 10 minutes. The lipid-dispersion is transferred into a conventional liposome extruder (e. g. Lipex 10 ml thermobarrel extruder) and repeatedly filtered through a membrane of defined pore size (100 nm Nucleopore® membrane). The resulting liposomes have a diameter of ± 150 nm and the width of the size distribution (as expressed in the polydispersity index) is 0.1.

The measurement of size (Zave) and polydispersity (PI) of the liposomes described within this example was performed by DLS (= Photon Correlation Spectroscopy = PCS) using a Zetamaster (Malvern Instruments, Herrenberg, Germany). The software used to process PCS data was version 1.4+ (Malvern Instruments, Herrenberg, Germany).

### Example 2

| | |
|---|---|
| 40 mgs | DOTAP, N-[1-(1-2,3-Dioleyloxy) propyl]-N,N,N-trimethyl-ammonium-chlorid) |
| 40 mgs | DOPC, 1,2-Dioleoyl-sn-Glycero-3-Phosphocholin |
| 10 ml | glucose 5% |

The powdered lipids are weighed into a vessel and transferred into the compounder as depicted in Fig. 3. The lipids are homogenized at room temperature using the novel apparatus relating to the invention, without using any solvent or solubilizer, by passing the mass 10 x through the extruder.
The obtained homogeneously mixed lipid-mass is dispersed by stirring in aqueous medium (Glucose 5%) and allowed to hydrate and swell for approximately 10 minutes. The lipid-dispersion is transferred into a conventional liposome extruder (e. g. Lipex 10 ml thermobarrel extruder) and repeatedly filtered through a membrane of defined pore size (100 nm Nucleopore® membrane). The resulting liposomes have a diameter of ± 160 nm and the width of the size distribution (as expressed in the polydispersity index) is 0.05.

The measurement of size (Zave) and polydispersity (PI) of the liposomes described within this example was performed by DLS (= Photon Correlation Spectroscopy = PCS) using a Zetamaster (Malvern Instruments, Herrenberg, Germany). The software used to process PCS data was version 1.4+ (Malvern Instruments, Herrenberg, Germany).
A polydispersity index (PI) of < 0.2 was chosen as a limit for a narrow size distribution.

### Example 3

| | |
|---|---|
| 8 g | egg-phosphatidyl-choline (Egg-PC) |
| 1000 ml | glucose 5% |

The powdered crude egg-PC is recirculated at room temperature for approximately 20 minutes using a MiniLab® extruder, (ThermoHaake, Karlsruhe, Germany), without using any solvent or solubilizer. The obtained lipid-mass is dispersed in aqueous medium (Glucose 5%) and allowed to hydrate and swell for approximately 20 minutes. The lipid-dispersion is transferred into a conventional liposome extruder (e. g. Lipex 100 ml extruder) and repeatedly filtered through a membrane of defined pore size (100 nm Nucleopore® membrane). The resulting liposomes have a diameter of ± 150 nm and the width of the size distribution (as expressed in the polydispersity index) is < 0.15.
The measurement of size (Zₐᵥₑ) and polydispersity (PI) of the liposomes described within this example was performed by DLS (= Photon Correlation Spectroscopy = PCS) using a Zetamaster (Malvern Instruments, Herrenberg, Germany). The software used to process PCS data was version 1.4+ (Malvern Instruments, Herrenberg, Germany).

See Table 6, Size and polydispersity index of liposomes prepared according to the Example 3.

### Example 4

| | |
|---|---|
| 4 g | DOTAP |
| 4 g | DOPC |
| 1000 ml | glucose 5% |

The powdered lipids are recirculated at room temperature for approximately 20 minutes using a MiniLab® extruder, (ThermoHaake, Karlsruhe, Germany), an apparatus relating to the invention, without using any solvent or solubilizer.
The obtained homogeneously mixed lipid-mass is dispersed in aqueous medium (Glucose 5%) and allowed to hydrate and swell for approximately 10 minutes. The lipid-dispersion is transferred into a conventional liposome extruder (e. g. Lipex 100 ml extruder) and repeatedly filtered through a membrane of defined pore size (100 nm Nucleopore® membrane). The resulting liposomes have a diameter of ± 170 nm an the width of the size distribution (as expressed in the polydispersity index) is < 0.25.

The measurement of size (Zₐᵥₑ) and polydispersity (PI) of the liposomes described within this example was performed by DLS (= Photon Correlation Spectroscopy = PCS) using a Zetamaster (Malvern Instruments, Herrenberg, Germany). The software used to process PCS data was version 1.4+ (Malvern Instruments, Herrenberg, Germany).

See Table 6, Size and polydispersity index of liposomes prepared according to the Example 4.

### Example 5

| | |
|---|---|
| 50 mol % | DOTAP labelled with DOPE-Rhodamine |
| 50 mol % | DOPC labelled with DOPE-NBD |
| q.s. | glucose 5% |

The powdered lipids are weighed into a vessel and transferred into the compounder as described within the invention and as depicted in Fig. 3. The lipids are homogenized at room temperature using the compounder, without using any solvent or solubilizer, by passing the mass through the extruder.
The obtained homogeneously mixed lipid-mass is dispersed in aqueous medium (Glucose 5%) and allowed to hydrate and swell for approximately 10 minutes. The lipid-dispersion is transferred into a conventional liposome extruder (e. g. Lipex 10 ml thermobarrel extruder) and repeatedly filtered through a membrane of defined pore size (100 nm Nucleopore® membrane).
The resulting liposomes have a diameter of ± 160 nm and the width of the size distribution (as expressed in the polydispersity index) is 0.15.
The degree of lipid mixing on molecular level can be monitored using the Fluorescence Resonance Energy Transfer (FRET) technique.
Fluorescence Resonance Energy Transfer (FRET) is an interaction between the excited states of electrons in two dye molecules. The strength of the interaction is depending on the distance of the molecules. Excitation from a donor dye molecule is transferred to an acceptor dye molecule if a proximity of typically 10 - 100 Å is given. A further primary condition for FRET is an overlap of the fluorescence emission spectrum of the donor molecule with the absorption spectrum of the acceptor molecule.
Thus, FRET is a valuable technique for investigating phenomena in liposome bilayers where proximities of lipid molecules and their distribution within the liposomal membrane can be studied when fluorescently labelled lipid molecules are incorporated in the lipid bilayers of liposomes.

Fluorescence intensities are directly related to the efficiency of energy transfer from the donor to the acceptor dye molecule. A low fluorescence intensity thus indicates a low level of lipid mixing of the fluorescently labelled lipids in the bilayers of the liposomes, as the probability of proximity of different lipid molecules is statistically low when mixing is incomplete.

The liposomes described within this example have been investigated by the FRET technique as described before and the fluorescence intensities obtained in these studies indicated a much higher level of lipid mixing due to the homogenization process by the novel apparatus, according to the invention, as compared to liposomes prepared without this preparation step.

The measurement of size (Zₐᵥₑ) and polydispersity (PI) of the liposomes described within this example was performed by DLS (= Photon Correlation Spectroscopy = PCS) using a Zetamaster (Malvern Instruments, Herrenberg, Germany). The software used to process PCS data was version 1.4+ (Malvern Instruments, Herrenberg, Germany).

### Example 6

| | |
|---|---|
| 50 mol % | DOTAP |
| 50 mol % | DOPC |
| 10 ml | glucose 5% |

The powdered lipids are weighed into a vessel and transferred into the compounder as depicted in Fig. 3. The lipids are homogenized at room temperature using the novel apparatus relating to the invention, without using any solvent or solubilizer, by passing the mass 3 x through the extruder.
The obtained homogeneously mixed lipid-mass is dispersed in aqueous medium (glucose 5%) and allowed to hydrate and swell for approximately 10 minutes. The lipid-dispersion is transferred into a conventional liposome extruder (e. g. Lipex 10 ml thermobarrel extruder) and repeatedly filtered through a membrane of defined pore size (100 nm Nucleopore® membrane).
The diameter of the resulting liposomes and the width of the size distribution (as expressed in the polydispersity index) is depicted in Table 1.
The obtained liposome dispersion was immediately sterilized by means of sterile filtration with 0.2 µm sterile filters. (Durapore® (PVDF) Fa. Millipore, Eschborn, Germany). The data for liposomes before and after sterile filtration is depicted in Table 1.
Samples were taken of all preparations for PCS measurements.

The measurement of size (Zₐᵥₑ) and polydispersity (PI) of the liposomes described within this example was performed by DLS (= Photon Correlation Spectroscopy = PCS) using a Zetamaster (Malvern Instruments, Herrenberg, Germany). The software used to process PCS data was version 1.4+ (Malvern Instruments, Herrenberg, Germany).

Stability data (shelf live stability of 6 month) for liposomes prepared according example 6 is shown in table 3.

### Example 7

| | |
|---|---|
| 50 mol % | DOTAP |
| 50 mol % | DOPC |
| q.s. | glucose 5% |

See table 2 for the detailed data of liposomes prepared according to this example.

### The liposomes have been prepared according the following preparation protocol:

### Batch I.): Compounding according to the invention

The powdered lipids are weighed into a vessel and transferred into the compounder as depicted in Fig. 3. The lipids are homogenized at room temperature using the novel apparatus relating to the invention, without using any solvent or solubilizer, by passing the mass 3 x through the extruder.
The obtained homogeneously mixed lipid-mass is dispersed in aqueous medium (glucose 5%) and allowed to hydrate and swell for approximately 10 minutes. The lipid-dispersion is transferred into a conventional liposome extruder (e. g. Lipex 10 ml thermobarrel extruder) and repeatedly filtered through a membrane of defined pore size (100 nm Nucleopore® membrane).

### Batch II.): High-pressure homogenization

The lyophilised lipids were dispersed in glucose 5% by manually shaking and 10 minutes allowed to swell and hydrate.
The obtained raw lipid dispersion was then 10 x homogenized at 200 bar homogenization pressure using an APV Gaulin Micron Lab 40 high pressure homogenizer. (APV-Gaulin, Lübeck, Germany).

### Batch III.): Ethanol-injection technique

The lipids first were dissolved in EtOH _{abs}. intending to obtain a clear lipid stock solution of app. 400 mM lipid concentration.
Then the solute was injected, under slight stirring, into the aqueous medium, (glucose 5%) using a 1 ml Hamilton Syringe (Hamilton Inc., Switzerland). Then, the obtained dispersion was extruded 10 x through 100 nm polycarbonate membranes (Osmonics Inc., Minnetonka, USA) using a Lipex 10 ml thermo-barrel extruder. (Lipex Inc., Vancouver, Canada).

### Batch IV.): Sonication

For the preparation of liposomes by sonication, lipids first were dissolved in chloroforme intending to obtain a clear lipid solution.
Then the solvent was removed by rotary evaporation forming a thin lipid film on the sides of a round bottom flask. The lipid film was thoroughly dried to remove residual organic solvent by placing the flask on a vacuum pump for 1 hour. Hydration of the dry lipid film was accomplished by adding the aqueous medium (glucose 5%) to the round bottom flask and agitating.
The obtained dispersion of MLV was sonified in short cycles for 10 minutes using a Branson sonifier.

### Batch V.): Film-method

The lipids first were dissolved in chloroforme intending to obtain a clear lipid solution.
Then the solvent was removed by rotary evaporation forming a thin lipid film on the sides of a round bottom flask. The lipid film was thoroughly dried to remove residual organic solvent by placing the flask on a vacuum pump for 1 hour. Hydration of the dry lipid film was accomplished by adding the aqueous medium (glucose 5%) to the round bottom flask and agitating.

### Batch VI.): Film-method + consecutive extrusion

The lipids first were dissolved in chloroforme intending to obtain a clear lipid solution.
Then the solvent was removed by rotary evaporation forming a thin lipid film on the sides of a round bottom flask. The lipid film was thoroughly dried to remove residual organic solvent by placing the flask on a vacuum pump for 1 hour. Hydration of the dry lipid film was accomplished by adding the aqueous medium (glucose 5%) to the round bottom flask and agitating.
Finally liposomes were extruded 10 x through 100 nm polycarbonate membranes (Osmonics Inc., Minnetonka, USA) using a Lipex 10 ml thermo-barrel extruder. (Lipex Inc., Vancouver, Canada).

The measurement of size (Zₐᵥₑ) and polydispersity (PI) of the liposomes described within this example was performed by DLS (= Photon Correlation Spectroscopy = PCS) using a Zetamaster (Malvern Instruments, Herrenberg, Germany). The software used to process PCS data was version 1.4+ (Malvern Instruments, Herrenberg, Germany).
A polydispersity index (PI) of < 0.2 was chosen as a limit for a narrow size distribution.

### Example 8

| | |
|---|---|
| 50 mol % | DOTAP |
| 45 mol % | DOPC |
| 5 mol% | Rhodamine-DOPE |
| q.s. | glucose 5% |

The powdered lipids are weighed into a vessel and transferred into the compounder as depicted in Fig. 3. The lipids are homogenized at room temperature using the novel apparatus relating to the invention, without using any solvent or solubilizer, by passing the mass 3 x through the extruder.
The obtained homogeneously mixed lipid-mass is dispersed in aqueous medium (glucose 5%) and allowed to hydrate and swell for approximately 10 minutes. The lipid-dispersion is transferred into a conventional liposome extruder (e. g. Lipex 10 ml thermobarrel extruder) and repeatedly filtered through a membrane of defined pore size (100 nm Nucleopore® membrane).
Samples were taken of all preparations for PCS measurements.
The measurement of size (Zₐᵥₑ) and polydispersity (PI) of the liposomes described within this example was performed by DLS (= Photon Correlation Spectroscopy = PCS) using a Zetamaster (Malvern Instruments, Herrenberg, Germany). The software used to process PCS data was version 1.4+ (Malvern Instruments, Herrenberg, Germany).
The resulting liposomes have a diameter of ± 155 nm and the width of the size distribution (as expressed in the polydispersity index) is ±0.15.

The lipid contents of the liposomes prepared according to example 8 were analysed using HPLC. The resulting data for loading efficiencies are given in table 4.

### Example 9

| | |
|---|---|
| 50 mol % | DOTAP labelled with DOPE-Rhodamine |
| 50 mol % | DOPC labelled with DOPE-NBD |
| q.s. | glucose 5% |

Results are set out in Table 5: loading efficiencies for liposomes produced as described in this example.

The liposomes have been prepared according the following preparation protocol:

### Batch a.): Compounding according to the invention

The powdered lipids are weighed into a vessel and transferred into the compounder as depicted in Fig. 3. The lipids are homogenized at room temperature using the novel apparatus relating to the invention, without using any solvent or solubilizer, by passing the mass 3 x through the extruder.
The obtained homogeneously mixed lipid-mass is dispersed in aqueous medium (glucose 5%) and allowed to hydrate and swell for approximately 10 minutes. The lipid-dispersion is transferred into a conventional liposome extruder (e. g. Lipex 10 ml thermobarrel extruder) and repeatedly filtered through a membrane of defined pore size (100 nm Nucleopore® membrane).

### Batch b.): High-pressure homogenization

The lyophilised lipids were dispersed in glucose 5% by manually shaking and 10 minutes allowed to swell and hydrate.
The obtained raw lipid dispersion was then 10 x homogenized at 200 bar homogenization pressure using an APV Gaulin Micron Lab 40 high pressure homogenizer. (APV-Gaulin, Lübeck, Germany).

### Batch c.): Ethanol-injection technique

The lipids first were dissolved in EtOH _{abs}. intending to obtain a clear lipid stock solution of app. 400 mM lipid concentration.
Then the solute was injected, under slight stirring, into the aqueous medium, (glucose 5%) using a 1 ml Hamilton Syringe (Hamilton Inc., Switzerland). Then, the obtained dispersion was extruded 10 x through 100 nm polycarbonate membranes (Osmonics Inc., Minnetonka, USA) using a Lipex 10 ml thermo-barrel extruder. (Lipex Inc., Vancouver, Canada).

### Batch d.): "lipid dispersion technique" + consecutive extrusion

The lyophilised lipids were dispersed in glucose 5% by manually shaking and 10 minutes allowed to swell and hydrate. The obtained raw lipid dispersion was then extruded 10 x through 100 nm polycarbonate membranes (Osmonics Inc., Minnetonka, USA) using a Lipex 10 ml thermo-barrel extruder. (Lipex Inc., Vancouver, Canada)

### Batch e.): Film-method + consecutive extrusion

The lipids first were dissolved in chloroforme intending to obtain a clear lipid solution.
Then the solvent was removed by rotary evaporation forming a thin lipid film on the sides of a round bottom flask. The lipid film was thoroughly dried to remove residual organic solvent by placing the flask on a vacuum pump for 1 hour. Hydration of the dry lipid film was accomplished by adding the aqueous medium (glucose 5%) to the round bottom flask and agitating.
Finally liposomes were extruded 10 x through 100 nm polycarbonate membranes (Osmonics Inc., Minnetonka, USA) using a Lipex 10 ml thermo-barrel extruder. (Lipex Inc., Vancouver, Canada).

The degree of lipid mixing on molecular level can be monitored using the Fluorescence Resonance Energy Transfer (FRET) technique.
Fluorescence Resonance Energy Transfer (FRET) is an interaction between the excited states of electrons in two dye molecules. The strength of the interaction is depending on the distance of the molecules. Excitation from a donor dye molecule is transferred to an acceptor dye molecule if a proximity of typically 10 - 100 Å is given. A further primary condition for FRET is an overlap of the fluorescence emission spectrum of the donor molecule with the absorption spectrum of the acceptor molecule.
Thus, FRET is a valuable technique for investigating phenomena in liposome bilayers where proximities of lipid molecules and their distribution within the liposomal membrane can be studied when fluorescently labelled lipid molecules are incorporated in the lipid bilayers of liposomes.
Fluorescence intensities are directly related to the efficiency of energy transfer from the donor to the acceptor dye molecule. A low fluorescence intensity thus indicates a low level of lipid mixing of the fluorescently labelled lipids in the bilayers of the liposomes, as the probability of proximity of different lipid molecules is statistically low when mixing is incomplete.

The liposomes described within this example have been investigated by the FRET technique as described before and the fluorescence intensities obtained in these studies indicated a much higher level of lipid mixing due to the homogenization process by the novel apparatus, according to the invention, as compared to liposomes prepared without this preparation step.

The measurement of size (Zₐᵥₑ) and polydispersity (PI) of the liposomes described within this example was performed by DLS (= Photon Correlation Spectroscopy = PCS) using a Zetamaster (Malvern Instruments, Herrenberg, Germany). The software used to process PCS data was version 1.4+ (Malvern Instruments, Herrenberg, Germany).
A polydispersity index (PI) of < 0.2 was chosen as a limit for a narrow size distribution.

The aforementioned detailed examples and descriptions are presented for illustrative purposes and are not limiting.

### Example 10

The different liposomal formulations are given below.

The powdered lipid(s) and the active ingredient camptothecin-lactone (CPT-lactone) or camptothecin-carboxylate (CPT-carboxylate) are weighed into a vessel and transferred into the compounder as depicted in Fig. 4. The lipid(s) are homogenized at room temperature using the novel apparatus relating to the invention, without using any solvent or solubilizer, by passing the mass 3 x through the extruder.

The obtained homogeneously mixed lipid-mass including the active ingredient camptothecin-lactone (CPT-lactone) or camptothecin-carboxylate (CPT-carboxylate) is dispersed in aqueous medium (trehalose 10 %) and allowed to hydrate and swell for approximately 10 minutes. The lipid-dispersion is transferred into a conventional filter extruder (e. g. Lipex 10 ml thermobarrel extruder) and repeatedly (3x) filtered through a membrane of defined pore size (200 nm Nucleopore® membrane), at a temperature of 4°C.
Samples were taken of all preparations for PCS measurements.

The measurement of size (Zₐᵥₑ) and polydispersity (PI) of the liposomes described within this example was performed by DLS (= Photon Correlation Spectroscopy = PCS) using a Zetasizer
HS 3000 (Malvern Instruments, Herrenberg, Germany). The software used to process PCS data was version 1.61 (Malvern Instruments, Herrenberg, Germany).
The resulting liposome diameters and width of the size distribution (as expressed by the polydispersity index) is given in Table 6.
The lipid contents of the liposomes prepared according to Example 10 were analysed using HPLC. The resulting data for loading efficiencies are given in Table 6.

### Example 11

### Liposome formulations prepared according to Example 10

### Batch a.)

| | |
|---|---|
| 95.2 mol % | DOTAP |
| 4.8 mol% | Camptothecin-carboxylate |
| q.s. | trehalose 10% |

Final concentration 15 mM

### Batch b.)

| | |
|---|---|
| 47.5 mol % | DOTAP |
| 47.5 mol % | DOPC |
| 5 mol% | Camptothecin-carboxylate |
| q.s. | trehalose 10% |
| Final concentration 15 mM | |

### Batch c.)

| | |
|---|---|
| 95.2 mol % | DOTAP |
| 4.8 mol% | Camptothecin-lactone |
| q.s. | trehalose 10% |
| Final concentration 15 mM | |

### Batch d.)

| | |
|---|---|
| 47.5 mol % | DOTAP |
| 47.5 mol % | DOPC |
| 5 mol% | Camptothecin-lactone |
| q.s. | trehalose 10% |
| Final concentration 15 mM | |

### Batch e.)

| | |
|---|---|
| 95.2 mol % | DOTAP |
| 4.8 mol% | Camptothecin-carboxylate |
| q.s. | trehalose 10% |
| Final concentration 15 mM | |

The preparation of all batches was performed according to the method in Example 10.

## Claims

**1.** A method for producing homogenous colloidal nanoparticles, comprising the steps of
e) extruding a composition comprising at least one amphiphilic component by means of a compounder,
f) dispersing the extruded composition of step a) in an aqueous medium,
g) optionally homogenizing the preparation of step b) at least once and/or
h) optionally sterile filtrating the preparation of step b) or c),
wherein optionally at least one active agent is present in the composition of step a) and/or in said aqueous medium of step b).

**1.** The method of claim 1, wherein said colloidal nanoparticles are selected from micelles, liposomes, lipid nanospheres, preferably from liposomes.

**2.** The method of claim 1 or 2, wherein said homogenous colloidal nanoparticles are **characterized by** having a FRET of between about 100 % to about 80 % of reference colloidal nanoparticles produced by the film method.

**3.** The method of any one of the claims 1 to 2, wherein said amphiphilic component is selected from fats, oils, waxes, sterols or lipids such as cholesterol or phospholipids, lysolipids, lysophospholipids, sphingolipids or pegylated lipids with a positive, negative or neutral net change.

**4.** The method of any one of the claims 1 to 3, wherein said amphiphilic component is a cationic lipid or a mixture of lipids, preferably at least one cationic lipid and optionally a neutral lipid.

**5.** The method of any one of the claims 1 to 4, wherein said colloidal nanoparticles have a polydispersity index (Pl) of below about 0.4, preferably of below about 0.2.

**6.** The method of any one of the claims 1 to 5, further comprising manufacturing a dietyry, cosmetic or pharmaceutical composition from the colloidal nanoparticles.

**7.** The method of any one of the claims 1 to 6, wherein said active agent is selected from biologically active agents such as dietary supplements, vitamins, cosmetics, diagnostic or therapeutic agents, preferably from diagnostic or therapeutic agents.

**8.** The method of any one of the claims 1 to 7, wherein step a) is performed without organic solvent and/or detergent.

**9.** The method of any one of the claims 1 to 8, wherein step a) is performed without an aqueous medium.

**10.** The method of any one of the claims 1 to 9, wherein the temperature of step a) is between about 5°C to about 100°C, preferably between about 20°C to 70°C.

**11.** The method of any one of the claims 1 to 10, wherein the pressure of step a) is between about 0,2 bar to about 100 bar, preferably about 0,5 bar to about 10 bar.

**12.** The method of any one of the claims 1 to 11, wherein said compounder is a batch extruder or a continuous extruder.

**13.** Use of a compounder comprising a cylinder and a plunger, wherein the cylinder has an open bore of about 0.1 mm to about 2 mm, preferably of about 0.2 mm to about 1.4 mm, more preferably of about 0.4 mm to about 1.2 mm and most preferably of about 0.8 mm at the lower end for the manufacture of colloidal nanoparticles, in a method according to any one of the claims 1 to 12.
**Table 1:**
| **Vesicle size and polydispersity index obtained when preparing liposomes according to the novel technique disclosed herein** | | | | |
|---|---|---|---|---|
| novel technique - using the compounder | liposome size [nm] | polydispersity index [PI] | liposome size [nm] after sterile filtration | Pl after sterile filtration |
| Batch-N°1 | 160 nm | 0.162 | 156 nm | 0.153 |
| Batch-N° 2 | 154 nm | 0.102 | 149 nm | 0.094 |
| Batch-N° 3 | 167 nm | 0.045 | 161 nm | 0.052 |
| Batch-N° 4 | 149 nm | 0.150 | 147 nm | 0.138 |
| Batch-N° 5 | 143 nm | 0.089 | 142 nm | 0.090 |
| Batch-N° 6 | 162 nm | 0.174 | 155 nm | 0.142 |
| Batch-N° 7 | 165 nm | 0.123 | 153 nm | 0.112 |
| Batch-N° 8 | 171 nm | 0.210 | 154 nm | 0.178 |
The preparation of Batch-N° 1 to 8 was performed according to example 6 (see above).
**Table 2:**
| **Vesicle size and polydispersity index obtained when preparing liposomes according to conventional preparation techniques and according to the novel technique disclosed herein** | | | | |
|---|---|---|---|---|
| mode of preparation | liposome size [nm] | polydispersity index [Pl] | liposome size [nm] after sterile filtration | Pl after sterile filtration |
| Batch I) novel technique - using the compounder | 154 nm | 0.056 | 149 nm | 0.065 |
| Batch II) high-pressure homogenization (APV Gaulin Lab 40) | 130 nm | 0.458 | 128 nm | 0.450 |
| Batch III) ethanol-injection-technique+consecutive extrusion | 136 nm | 0.332 | 131 nm | 0.324 |
| Batch IV) sonication (e.g using a Branson sonifier) | 142 nm | 0.381 | 140 nm | 0.354 |
| Batch V) Batch V) film-method | 987 nm | 0.890 | 197 nm | 0.226 |
| Batch VI) film-method and consecutive extrusion | 152 nm | 0.071 | 148 nm | 0.063 |
Preparation of liposomes performed according the description in example 7.
**Table 3:**
| **Physical stability studies of liposomes prepared according to the novel technique disclosed herein** | | | | |
|---|---|---|---|---|
| novel technique - using the compounder | vesicle size [nm] t = 0 | polydispersity index [PI] t = 0 | vesicle size [nm] t = 6 month | polydispersity index [PI] t = 6 month |
| Batch-N° 1 | 160 nm | 0.162 | 164 nm | 0.133 |
| Batch-N° 2 | 154 nm | 0.102 | 161 nm | 0.108 |
| Batch-N° 3 | 167 nm | 0.045 | 170 nm | 0.110 |
| Batch-N° 4 | 149 nm | 0.150 | 152 nm | 0.176 |
| Batch-N° 5 | 143 nm | 0.089 | 139 nm | 0.141 |
| Batch-N° 6 | 162 nm | 0.174 | 168nm | 0.203 |
| Batch-N° 7 | 165 nm | 0.123 | 166 nm | 0.146 |
| Batch-N° 8 | 171 nm | 0.210 | 175 nm | 0.182 |
The preparation of Batch-N° 1 to 8 was performed according to example 6.
**Table 4:**
| **Loading efficiencies for liposomes prepared according to the novel technique disclosed herein** | | | |
|---|---|---|---|
| | mean mol% cationic lipid * | mean mol% neutral co lipid * | mean mol% active agent * |
| Batch-N° a ** | 48.253 | 46.685 | 5.050 |
| Batch-N° b ** | 51.904 | 43.802 | 4.645 |
| Batch-N° c** | 49.391 | 47.269 | 5.756 |
| Batch-N° d** | 47.036 | 48.298 | 4.417 |
| film-method and consecutive extrusion | 49.495 | 45.579 | 4.926 |
| | | | |
|---|---|---|---|
| * values represent the average of duplicates | | | |
| ** all liposomes prepared according example 8 | | | |
**Table 5:**
| **FRET data obtained by investigating liposomes prepared according to the invention and according to conventional preparation techniques** | | | |
|---|---|---|---|
| mode of preparation | liposome size [nm] | polydispersity index [PI] | relative FRET in % |
| Batch a.): novel technique - using the compounder | 154 nm | 0.056 | 97.28 % |
| Batch b.): high-pressure-homogenization (e.g. using APV Gaulin Lab 40) | 130 nm | 0.458 | 31.45% |
| Batch c.): ethanol-injection-technique and consecutive extrusion | 136 nm | 0.332 | 69.97 % |
| Batch d.): lipid-dispersion-method and consecutive extrusion | 163 nm | 0.140 | 55.10 % |
| Batch e.): film-method and consecutive extrusion | 152 nm | 0.071 | 100 % |
Preparation of liposomes depicted in Table 5 performed according to example 7.
**Table 6:**
| **Vesicle size and polydispersity index obtained when preparing liposomes according to the novel technique disclosed herein** | | |
|---|---|---|
| **Liposome formulation + Novel technique - using the compounder** | **liposome size [nm] after extrusion 200 nm** | **polydispersity index [PI] after extrusion 200 nm** |
| Batch a.): DOTAP/CPT-carboxylate 15 mM | 177.1 nm | 0.24 |
| Batch b.): DOTAP/DOPC/CPT-carboxylate 15 mM | 185.9 nm | 0.26 |
| Batch c.): DOTAP/CPT-lactone 15 mM | 163.5 nm | 0.34 |
| Batch d.): DOTAP/DOPC/CPT-lactone 15 mM | 172.3 nm | 0.35 |
| Batch e.): DOTAP/CPT-carboxylate Extrusion with 100 nm PC-membrane 15 mM | 142.0 nm | 0.17 |
The preparation of Batches a.) to e.) was performed according to Example 10 (see above).
**Table 7:**
| **Results from HPLC quantification of CPT** | | | | |
|---|---|---|---|---|
| **Batch** | **Composition** | **conc. CPT total (µM)** | **conc. CPT before extrusion (µM)** | **conc. CPT after Extrusion (µM)** |
| a | DOTAP/CPT-Carboxylate | 814 | 949 | 938 |
| b | DOTAP/DOPC/CPT-Carboxylate | 702 | 932 | 932 |
| c | DOTAP/CPT-Lactone | 886 | 808 | 60 |
| d | DOTAP/DOPC/CPT-Lactone | 804 | 809 | 69 |
